# FASCICULE DE BREVET EUROPEEN

(11) **EP 2 591 350 B1**
(45) Date de publication et mention de la délivrance du brevet: **26.10.2016**
(21) Numéro de dépôt: 11743099.1
(22) Date de dépôt: 05.07.2011
(51) Int. Cl.: A61K 9/00, G01N 31/22, A61K 9/20, A61K 9/50, A61K 47/00

(54) **MÉTHODE ET AGENT POUR LA DÉTECTION DE DROGUES DANS LES BOISSON**
VERFAHREN UND AGENS ZUM NACHWEIS VON DROGEN IN GETRÄNKEN
METHOD AND AGENT FOR DETECTING DRUGS IN BEVERAGES

(30) Priorité: 06.07.2010 FR 1055490
(43) Date de publication de la demande: 15.05.2013
(73) Titulaire: ETHYPHARM, 92213 Saint-Cloud Cedex (FR)
(72) Inventeur: HERRY, Catherine, F-27670 Saint-Ouen du Tilleul (FR); CONTAMIN, Pauline, F-76220 La Feuillie (FR); DUPAU, Emmanuel, F-76100 Rouen (FR)
(74) Mandataire: Cabinet Plasseraud
(86) Numéro de dépôt international: PCT/FR2011/051600
(87) Numéro de publication internationale: WO 2012/010765

(56) Documents cités:
- EP-A1- 0 923 934
- EP-A2- 1 273 301
- WO-A1-00/38649
- WO-A2-03/021254
- WO-A2-03/026621
- FR-A1- 2 829 932
- GB-A- 1 157 662
- US-B1- 6 514 531
- US-B1- 6 617 123
- MOFFAT ET AL: "Drugs of abuse", SCIENCE AND JUSTICE, HARROGATE, GB, vol. 40, no. 2, 1 April 2000 (2000-04-01), pages 89-92, XP022547589, ISSN: 1355-0306, DOI: 10.1016/S1355-0306(00)71949-6

## Description

L'invention a pour objet une méthode pour lutter contre la soumission chimique.

Depuis un certain nombre d'années les délinquants utilisent les propriétés hypnotiques de certaines molécules pour droguer quelqu'un à son insu. Les malfaiteurs n'hésitent pas à introduire subrepticement dans la boisson de leur victime une forme pharmaceutique pour altérer le comportement de celle-ci, voire la rendre totalement amnésique. Une fois la victime démunie de toute conscience, le malfaiteur peut profiter d'elle : vol, viol, extorsion de fonds. Par ailleurs, l'ingestion d'une telle forme pharmaceutique sans respect des doses prescrites peut engendrer de sérieuses conséquences, notamment si elle est absorbée avec une quantité d'alcool. La forme pharmaceutique peut également produire des interactions délétères avec d'autres médicaments que la victime aurait pris au préalable.

Il est connu de l'état de la technique un somnifère, le Rohypnol, largement utilisé à des fins illicites en raison de sa facilité de dissolution et de ses caractéristiques imperceptibles. La formulation de ce médicament a été révisée pour aboutir à un comprimé vert à l'extérieur et bleu à l'intérieur, pelliculé donc lent à fondre, dégageant une couleur bleu. Cependant, la coloration bleue n'est visible qu'après un quart d'heure d'immersion dans le liquide ; la victime n'est donc pas en mesure de détecter le somnifère introduit subrepticement si elle boit immédiatement sa boisson.

Le document WO 03/026621 décrit un produit pharmaceutique administrable par voie orale comprenant, outre le principe actif sensible, au moins un agent fluorescent et au moins un agent détrompeur de goût, constitué notamment d'une substance amère, ou de particules insolubles ou de globules gélifiés aisément reconnaissables en bouche.

Le document WO 00/38649 décrit notamment un comprimé pharmaceutique pour l'administration par voie orale doté de moyens visuels qui se mettent en place après introduction dans une boisson éventuellement alcoolisée, consistant en la flottaison dudit comprimé et/ou en la formation de particules insolubles.

Il est également connu de l'état de la technique le document WO 2005/059541 qui porte sur un kit pour détecter les drogues introduites furtivement dans les boissons. Toutefois, ce système ne protège que les personnes munies de ce kit.

Il est donc impératif et urgent de trouver une méthode permettant de détecter immédiatement le détournement illicite de médicaments en cas de soumission chimique sans avoir recours à un dispositif ou à un kit de détection.

Un objectif essentiel de la présente invention est donc une telle méthode mettant en oeuvre une forme pharmaceutique comprenant au moins un composé permettant la détection immédiate de ladite forme pharmaceutique introduite illicitement dans une boisson. L'invention porte également sur cette forme pharmaceutique.

Le but de la présente invention est d'offrir une nouvelle méthode pour lutter contre la soumission chimique. Ce but est atteint grâce à une méthode pour lutter contre la soumission chimique telle que définie dans les revendications annexées. La méthode décrite comprend:
- la mise en solution dans une boisson d'une forme pharmaceutique solide comportant un principe actif et au moins 0,05 mg, de préférence de 0,2 à 5 mg, plus préférentiellement encore de 0,3 à 2 mg d'au moins un agent colorant hydrosoluble acceptable par voie orale choisi dans le groupe comprenant l'indigocarmine, l'érythrosine, le brillant bleu FCF, l'alphazurine FG, le fast green FCF, la quinzarine green SS, l'orange II, la tartrazine, Sunset yellow FCF,
- la détection de la forme pharmaceutique par la modification immédiate de la couleur de la boisson.

Est également décrite l'utilisation dans une forme pharmaceutique solide, d'au moins 0,05 mg, de préférence de 0,2 à 5 mg, plus préférentiellement encore de 0,3 à 2mg d'au moins un agent colorant hydrosoluble choisi parmi l'indigocarmine, l'érythrosine, le brillant bleu FCF, l'alphazurine FG, le fast green FCF, la quinzarine green SS, l'orange II, la tartrazine, Sunset yellow FCF.pour lutter contre la soumission chimique.

L'invention porte également sur une forme pharmaceutique solide non pelliculée telle que définie dans les revendications annexées, pour lutter contre la soumission chimique. Elle comprend un principe actif et au moins 0,05 mg, de préférence de 0,2 à 5 mg, plus préférentiellement encore de 0,3 à 2 mg d'au moins un agent colorant hydrosoluble choisi parmi l'indigocarmine, l'érythrosine, le brillant bleu FCF, l'alphazurine FG, le fast green FCF, la quinzarine green SS, l'orange II, la tartrazine, Sunset yellow FCF, et des excipients pharmaceutiquement acceptables.

Dans la présente invention, par "forme pharmaceutique solide non pelliculée", on entend toute forme solide qui ne comprend pas d'enrobage sur sa surface la plus externe destinée à être en contact avec le milieu dans lequel elle est dissoute ou avec les muqueuses. Ainsi, dans le cas d'un comprimé, celui-ci ne comportera pas de pelliculage externe. Pour autant, la substance active pourra être présente au sein de ce comprimé dans ou sous la forme de granules enrobés.

Par « soumission chimique » on entend l'administration à des fins criminelles ou délictuelles d'une substance psychoactive à l'insu de la victime.

### DESCRIPTION DETAILLEE DE L'INVENTION

Selon un premier objet, la présente invention porte sur une méthode permettant la détection immédiate d'un principe actif qui modifie l'état de conscience d'une victime introduit illicitement dans une boisson, ladite méthode étant telle que définie à l'une quelconque des revendications 1 à 5.

Selon un autre objet, l'invention porte sur une forme pharmaceutique solide non pelliculée permettant la détection immédiate d'un principe actif qui modifie l'état de conscience d'une victime introduit illicitement dans une boisson, ladite forme pharmaceutique étant telle que définie à l'une quelconque des revendications 6 à 8.

La description détaillée des différents éléments constituants l'invention s'applique de façon indifférente à tous les objets de l'invention : procédé composition.

La forme pharmaceutique solide selon l'invention comprend un principe actif et au moins un agent colorant hydrosoluble, qui permet la détection immédiate de ladite forme pharmaceutique introduite illicitement dans une boisson.

L'agent colorant intégré dans une forme pharmaceutique permet de colorer la boisson dans laquelle la forme pharmaceutique est introduite. Cet agent est particulièrement intéressant car il permet de colorer tous les types de boisson, qu'elles soient limpides ou opaques à l'exception des boissons très foncées comme le café ou le coca.

### Les agents colorants hydrosolubles

Selon un premier aspect de l'invention, les agents colorants hydrosolubles pouvant être mis en oeuvre dans l'invention sont des colorants solubles dans tout liquide comprenant au moins en partie de l'eau et qui sont pharmaceutiquement acceptables. De tels agents colorants sont choisis dans le groupe suivant : l'indigocarmine ou E 132, l'érythrosine ou E 127, le brillant bleu FCF, l'alphazurine FG, le fast green FCF, la quinzarine green SS, l'orange II, la tartrazine, Sunset yellow FCF.

L'agent colorant est présent en une quantité suffisante pour permettre une coloration assez intense pour être perçue à l'oeil nu et qui puisse apparaître dès les premières secondes après l'introduction de la forme pharmaceutique dans ladite boisson. Ainsi, l'agent colorant est présent à raison d'au moins 0,05 mg, de préférence de 0,2 à 5 mg, plus préférentiellement encore de 0,3 à 2 mg dans la forme pharmaceutique.

Lorsque l'agent colorant est de l'indigocarmine, une couleur bleu se dégage immédiatement de la forme pharmaceutique, colorant par exemple la boisson en bleu s'il s'agit d'une boisson incolore comme l'eau ou la limonade, ou en vert s'il s'agit d'une boisson jaune comme le jus d'orange.

L'érythrosine, autre agent colorant, colore les boissons en rouge.

Dans la présente invention, on entend par "immédiate" la modification des caractéristiques organoleptiques de la boisson qui se produit en moins d'une minute, de préférence en moins de 30 secondes, plus préférentiellement encore en moins de 15 secondes à partir de l'introduction de la forme pharmaceutique dans la boisson. Par exemple, dans le cadre de la présente invention, l'apparition ou la modification de la couleur peut se produire en moins de 30 secondes, de préférence en moins de 15 secondes.

Selon un autre aspect, le terme "immédiate" peut également être défini comme la modification des caractéristiques organoleptiques de la boisson qui se produit en moins d'une minute, de préférence en moins de 30 secondes, plus préférentiellement encore en moins de 15 secondes à partir de l'introduction et de l'agitation de la forme pharmaceutique dans la boisson.

On entend par « agitation » une mise en mouvement du liquide, par exemple à l'aide d'une paille, cuillère, ou par mouvement du récipient.

Selon différents exemples et outre les agents colorants qui sont décrits plus haut et qui permettent de colorer une boisson piégée, la détection de la substance active dans la boisson peut en plus se faire par la présence dans la forme pharmaceutique solide d'au moins un composé choisi dans le groupe comprenant :
- des agents opacifiants, et/ou
- des agents fluorescents, et/ou
- des particules flottantes, et/ou
- des particules perceptibles en bouche, et/ou
- des microgranules effervescents.

Lesdits composés peuvent être intégrés à la forme pharmaceutique solide individuellement ou en combinaison. On pourra par exemple réaliser une forme pharmaceutique contenant un agent colorant associé à des particules flottantes, ou bien proposer une forme pharmaceutique solide comprenant un agent colorant et un mélange des composés décrits ci-dessus.

Avantageusement, la forme pharmaceutique solide comprend au moins un agent colorant avec des particules flottantes et/ou des particules perceptibles en bouche et/ou des microgranules effervescents.

### Les agents opacifiants

Les agents opacifiants sont des composés minéraux qui permettent de troubler les boissons. Il peut s'agir de silicates tels que le silicate de magnésium, le silicate d'aluminium (notamment le kaolin), le silicate de magnésium et d'aluminium, le silicate de calcium, le dioxyde de titane et leurs mélanges. Ces composés sont généralement présents en une quantité d'au moins 15 mg, de préférence de 15 à 100 mg, préférentiellement encore de 20 mg à 60 mg et plus préférentiellement encore de 25 à 40 mg. En dessous de 15 mg, l'opacité pourrait s'avérer plus difficilement détectable à l'oeil nu.

Les agents opacifiants vont permettre de troubler les boissons dans lesquelles la forme pharmaceutique est introduite. Ainsi, non seulement la boisson changera de couleur mais deviendra trouble ce qui attirera davantage l'attention de la personne à qui cette boisson est destinée et facilitera ainsi la détection d'une substance active non désirée.

Ces agents sont particulièrement intéressants pour troubler les boissons transparentes et claires comme l'eau, le vin blanc, le jus de pomme, les spiritueux tels que la vodka, le rhum blanc....

L'aspect opaque de la boisson apparait dès les premières secondes après l'introduction et de préférence l'agitation de la forme pharmaceutique dans ladite boisson, concomitamment avec la modification de la couleur.

### Les agents fluorescents

La forme pharmaceutique solide peut également comprendre un agent fluorescent en une quantité d'au moins 0,1 mg, de préférence en une quantité d'au moins 1 mg, préférentiellement encore entre 0,2 et 5 mg et plus préférentiellement encore entre 0,3 à 2 mg. Cet agent peut être la fluorescéine et ses dérivés, le vert d'indocyanine.

Cet agent est visible dans tous types de boisson en présence de rayonnements ultra-violets et dans l'obscurité. Il permet de révéler la forme pharmaceutique le contenant en émettant une lumière fluorescente qui se dégage de la boisson piégée. Cet agent est particulièrement utile pour avertir la victime lorsqu'elle se trouve dans un espace sombre où il est facile d'introduire furtivement un corps étranger dans une boisson.

Avantageusement, la boisson piégée devenue fluorescente parait plus lumineuse qu'une boisson piégée contenant une forme pharmaceutique avec agent colorant mais dépourvue d'agent fluorescent permettant ainsi d'alerter immédiatement la victime.

### Les particules flottantes et les particules perceptibles en bouche

La forme pharmaceutique solide peut comprendre des particules flottantes et/ou des particules perceptibles en bouche. Ces particules sont des microgranules comprenant un support neutre insoluble, ou rendu insoluble dans l'eau ou dans une solution alcoolique par enrobage avec un polymère insoluble ou par enrobage d'une matière lipidique.

### Microgranules

On entend par microgranules rendus insolubles dans l'eau ou dans une solution alcoolique, un support neutre constitué par des matériaux solubles dans l'eau ou dans une solution alcoolique recouverts d'au moins une couche de matériaux insolubles dans l'eau ou dans une solution alcoolique et dont la fonction est de limiter, voire d'empêcher la pénétration de ces dits milieux vers le coeur du support.

De façon avantageuse, le support neutre insoluble dans l'eau ou dans une solution alcoolique comprend au moins un excipient de nature hydrophobe choisi parmi : la cellulose, les dérivés de la cellulose (cellulose microcristalline), les dérivés des phosphates (phosphates de calcium), la silice et les dérivés des silicates (silicate de magnésium, silicate d'aluminium et leurs mélanges), la cire de carnauba.

Dans le cadre de la présente invention, un support neutre soluble dans l'eau ou dans une solution alcoolique peut également être utilisé. Le support neutre soluble peut comprendre au moins un excipient choisi parmi : l'amidon, le saccharose, les polyols tels que le mannitol ou le lactose et leurs mélanges.

Il est impératif que ce support neutre soluble soit rendu insoluble dans l'eau ou l'alcool en le recouvrant d'une couche d'enrobage de nature :
- soit polymérique comprenant au moins un polymère hydrophobe et éventuellement une charge inerte et/ou un agent plastifiant et/ou un agent tensio-actif,
- soit lipidique comprenant au moins une matière lipidique.

Dans le cadre de la présente invention, le support neutre insoluble peut également être recouvert par au moins une couche d'enrobage telle que décrite ci-dessus, dans la mesure où celle-ci n'augmente pas de façon rédhibitoire la densité des particules.

Le taux d'enrobage représente le rapport de la quantité de masse sèche constituant la couche d'enrobage sur la masse totale du microgranule avant enrobage (en masse sèche). Le taux d'enrobage est compris entre 0,1% à 50% m/m, de préférence de 2% à 30% m/m, et, plus préférentiellement encore de 5% à 40% m/m.

Le taux d'enrobage est tel que les particules obtenues ont une densité inférieure à celle de la boisson dans laquelle elles vont être introduites, de préférence une densité inférieure à 1, de telle sorte qu'elles restent à la surface de la boisson dans laquelle elles vont être introduites. De telles particules sont appelées particules flottantes.

### Couche d'enrobage polymérique :

Le polymère hydrophobe utilisé pour assurer le caractère insoluble des microgranules est sélectionné dans le groupe des produits suivants : les dérivés non hydrosolubles de la cellulose, les dérivés de (co)polymères (méth)acryliques, les dérivés des polyvinylacétates et leurs mélanges. Plus préférentiellement, le ou les polymère(s) hydrophobe(s) est (sont) choisi(s) dans le groupe de produits suivants : l'éthylcellulose, l'acétate butyrate de cellulose, l'acétate de cellulose, les copolymères ammonio-méthacrylates type A et type B vendus sous le nom commercial Eudragit®, notamment l'Eudragit® RS 30D, l'Eudragit NE 30D, l'Eudragit® RL 30D, l'Eudragit® RS PO et l'Eudragit® RL PO de la famille des poly(éthyl acrylate, méthyl méthacrylate, triméthylamonioéthyl méthacrylate), les polyvinylacétates et leurs mélanges.

La quantité de polymère hydrophobe est comprise entre 50% à 100%, de préférence de 70% à 100%, de la masse sèche de la couche d'enrobage.

Une charge inerte peut être présente dans la couche d'enrobage à raison de 0 à 50% m/m, de préférence de 0 à 20% m/m, et, plus préférentiellement encore, de 5 à 20% de la masse sèche du polymère hydrophobe d'enrobage.

La charge inerte uniformément répartie dans l'enrobage est choisie dans le groupe comprenant notamment le talc, la silice colloïdale anhydre, le stéarate de magnésium, le monostéarate de glycérol et leurs mélanges.

Lorsque l'enrobage est réalisé par voie aqueuse, un agent plastifiant peut être ajouté à la dispersion d'enrobage à raison de 0% à 50% m/m, de préférence, de 2% à 25% m/m, en masse sèche de polymère hydrophobe d'enrobage.

L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-mono-stéarate, glycéryl-triacétate, glycéryl-tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétyltriéthylcitrate, tributylcitrate, triéthylcitrate, les sébaçates, de préférence dans le sous-groupe suivant : diéthylsébaçate, dibutylsébaçate, les adipates, les azélates, les benzoates, le chlorobutanol, les polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin (celle-ci étant particulièrement préférée), et leurs mélanges.

Plus préférentiellement, l'agent plastifiant est sélectionné dans le groupe de produits suivants : les monoglycérides acétylés notamment le Myvacet® 9-45, le triéthylcitrate (TEC), le dibutylsébacate, la triacétine et leurs mélanges.

L'agent tensioactif est optionnellement présent dans l'enrobage à raison de 0 à 30% m/m, de préférence de 0 à 20% m/m, et, plus préférentiellement encore, de 5 à 15% de la masse sèche de plastifiant. L'agent tensioactif est de préférence sélectionné dans le groupe de produits suivants : les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

### Couche d'enrobage lipidique :

Les microgranules peuvent également être enrobés par enrobage d'une matière lipidique.
La matière lipidique est sélectionnée notamment dans le groupe de produits suivants : glycéryl palmitostéarate, les cires, les polyoxylglycérides, le glycéryl béhénate.

La quantité de matière lipidique est comprise entre 50 et 100 %, de préférence entre 80 et 100%, de la masse sèche de la couche d'enrobage.
La quantité de matière lipidique est choisie de telle sorte que la densité des particules résultantes soit inférieure à celle de la boisson dans laquelle elles vont être introduites, de préférence une densité inférieure à 1, de telle sorte qu'elles restent à la surface de la boisson dans laquelle elles vont être introduites.

Les particules flottantes présentent un diamètre total (support neutre optionnellement enrobé si besoin,) compris entre 50 et 500 µm, de préférence entre 200 et 500 µm afin de ne pas être perceptibles en bouche et assurer un certain confort pour le patient. En revanche, les particules perceptibles en bouche présentent quant à elles un diamètre total supérieur à 500.µm, de préférence supérieur à 1 mm de façon à être perçues par les lèvres et surtout par les papilles. La mesure du diamètre des particules flottantes et perceptibles en bouche est faite par granulométrie laser en voie sèche (granulomètre laser Malvern : mastersizer 2000).

De façon tout à fait avantageuse, lesdites particules perceptibles en bouche sont flottantes.

La quantité des particules flottantes et/ou perceptibles en bouche contenues dans la forme pharmaceutique est d'au moins 25 mg, de préférence 40 mg.

De façon préférée, les particules flottantes et/ou les particules perceptibles en bouche peuvent être colorées à l'aide d'au moins un agent colorant suivant : l'indigocarmine, l'érythrosine, le brillant bleu FCF, l'alphazurine FG, le fast green FCF, la quinzarine green SS, l'orange II, la tartrazine, Sunset yellow FCF et/ou peuvent être rendus fluorescents à l'aide d'un agent fluorescent choisi dans le groupe comprenant la fluorescéine et ses dérivés, le vert d'indocyanine.

Avantageusement, le principe actif pourra également être coloré avec au moins un colorant tel que décrit ci-dessus afin d'empêcher le tri possible entre le principe actif et les particules flottantes et/ou perceptibles en bouche.

Avantageusement encore, les particules flottantes et perceptibles en bouche s'adaptent à tous types de boisson.

Dès l'introduction de la forme pharmaceutique dans la boisson, les particules flottantes remontent immédiatement à la surface de la boisson et sont visibles à l'oeil nu. Ces particules restent à la surface du liquide pendant au moins 5 minutes et de façon préférée pendant au moins 4 heures, plus préférentiellement pendant au moins 12 heures.

Les particules perceptibles en bouche, peuvent également être des particules flottantes. Elles sont quant à elles, détectées immédiatement par la victime lors de la prise de la première gorgée de la boisson piégée.

### Les microgranules effervescents

La forme pharmaceutique solide peut également comprendre des microgranules effervescents. Les microgranules effervescents comprennent un excipient basique qui créera une effervescence lorsqu'il sera en présence d'une boisson acide de type soda ou bière.

Selon un premier aspect, les microgranules comprennent un support neutre (soluble, insoluble ou rendu insoluble) enrobé par des particules d'un agent alcalin choisi dans le groupe comprenant le bicarbonate de sodium, le carbonate de calcium, ou leurs mélanges.

La quantité d'agent alcalin est au moins supérieure à 5 mg, de préférence supérieure à 10 mg et encore plus préférentiellement supérieure à 20 mg.

Lorsque la forme pharmaceutique contenant les microgranules effervescents est introduite dans une boisson acide, les particules d'agent(s) alcalin(s) au contact de l'acide présent créent une effervescence visible à l'oeil nu.

Selon un exemple les microgranules effervescents peuvent être enrobés. L'enrobage est suffisamment perméable pour permettre la libération des particules d'agent effervescent sur une période d'au moins trente minutes à une heure. L'enrobage contient au moins un polymère insoluble de la famille des dérivés de la cellulose, des dérivés vinyliques ou des dérivés acryliques. Il peut comprendre un plastifiant et/ou un agent tensioactif. Il peut être perméabilisé par ajout d'un agent porogène soluble comme par exemple des dérivés solubles de la cellulose, la povidone, un agent désintégrant.

La quantité de microgranules effervescents contenus dans la forme pharmaceutique est d'au moins 25 mg, de préférence 40 mg.

De façon préférée, les microgranules effervescents peuvent être colorés à l'aide d'au moins un agent colorant choisi parmi l'indigocarmine, l'érythrosine, le brillant bleu FCF, l'alphazurine FG, le fast green FCF, la quinzarine green SS, l'orange II, la tartrazine, Sunset yellow FCF et/ou peuvent être rendus fluorescents à l'aide d'un agent fluorescent choisi dans le groupe comprenant la fluorescéine et ses dérivés, le vert d'indocyanine.

Ainsi, une effervescence apparaitra à la surface de la boisson après introduction de la forme pharmaceutique contenant lesdits microgranules.

### Le principe actif

L'invention est adaptée pour tout principe actif qui modifie l'état de conscience du patient. Plus particulièrement, le principe actif est choisi dans le groupe comprenant : les anxyolytiques par exemple les benzodiazépines, les hypnotiques, les sédatifs, les analgésiques par exemple de type opioïde.

Les anxyolytiques sont une classe de médicaments psychotropes, de préférence choisi parmi l'Alprazolam, le Bromazépam, le Chlordiazépoxide, le Clobazam, le Clonazépam, le Clotiazépam, le Clorazépate, le Diazépam, l'Estazolam, le Flunitrazépam, le Lorazépam, le Lormétazépam, le Midazolam, le Nitrazépam, le Nordazépam, l'Oxazépam, le Prazépam, le Témazépam, le Tétrazépam, le Triazolam, la clozapine, l'olanzapine, la pirenzépine, le zolpidem, lezopiclone, le zaléplon, le méprobamate, l'étifoxine.

Les opioïdes sont de préférence choisi parmi l'Alfentanil, l'Aniléridine, le Butorphanol, le carfentanil, la Codéine, la Diamorphine (héroine), le Dextropropoxyphène, les Enképhalines, les Endorphines, le Fentanyl, l'Hydrocodone, l'Hydromorphone, la Méthadone, la Morphine, la Nalbuphine, l'Oxycodone, l'Oxymorphone, la Pentazocine, la Péthidine (mépéridine), le Propoxyphène, le Rémifentanil, la Sufentanil, le Tramadol et la Buprénorphine.

Selon un aspect particulier de l'invention, le principe actif présent dans la forme pharmaceutique est sous forme solide.

Selon un mode de réalisation particulier, le principe actif pourra également être coloré à l'aide d'au moins un agent colorant. L'agent colorant peut être l'un de ceux décrits précédemment et/ou pourra être rendu fluorescent par ajout d'un agent fluorescent tel que décrit précédemment.

Selon un autre mode de réalisation, le principe actif pourra être monté sur les particules flottantes et/ou sur les particules perceptibles en bouche.

### Boisson

Dans la présente demande, on utilisera le terme boisson pour désigner les boissons froides et les boissons chaudes, par exemple l'eau ; l'eau pétillante ; le vin (rouge, blanc, rosé) ; la bière (brune, blonde) ; les liqueurs ; les spiritueux tels que la vodka, le rhum, l'eau-de-vie, la téquila, le whisky ; les cocktails ; les jus de fruits tels que le jus d'orange, le jus de raisin ; les sodas tel que le coca, la limonade ; le café ; le thé, la tisane. Ces boissons sont données à titre indicatif mais aucunement à titre limitatif.

Dans la présente invention, le récipient, contenant une boisson dans laquelle la forme pharmaceutique peut être introduite, a une contenance comprise entre 3 cl et 1 L.

### Le procédé de préparation

En fonction de sa nature, le principe actif peut être sous forme de microcristaux, de microgranules ou mis en suspension et monté sur un support neutre.

Lorsqu'il est monté sur un support neutre, le principe actif se trouve sous forme de solution ou suspension dans un solvant aqueux ou organique. Un agent liant, un diluant, un agent antistatique, peuvent également être ajoutés.

Le support neutre peut être tout excipient inerte chimiquement et pharmaceutiquement, existant sous forme particulaire, cristallin ou amorphe. On cite à titre d'exemple des dérivés de sucres tels que le lactose, le saccharose, l'amidon hydrolysé (maltodextrines) ou encore des celluloses. Des mélanges tels que le saccharose et l'amidon ou à base de cellulose sont également utilisé pour la préparation de supports neutres sphériques.

Le principe actif peut également être mis sous forme de microgranules par un procédé en lui-même connu tel que, par exemple, l'extrusion-sphéronisation, le montage de principe actif en turbine perforée, en lit fluidisé et autres.

Les différents procédés de fabrication des microgranules par granulation par voie sèche ou par voie humide, présentés dans « Remington's pharmaceutical Sciences, 16ème Ed, 1980, Mack Publ. Co. of Easton, PA, USA,» peuvent être mis en oeuvre dans la présente invention

Le principe actif peut être enrobé par un polymère choisi en fonction du type de libération souhaitée ( immédiate, contrôlée, retardée) ou en fonction de ses propriétés de masquage de goût.

Le principe actif est ensuite combiné avec au moins un colorant, éventuellement avec un autre composé permettant de lutter contre la soumission chimique, et avec au moins un excipient pharmaceutiquement acceptable.

L'invention s'adapte à toute forme pharmaceutique. Selon un aspect avantageux est décrit une composition pharmaceutique solide non pelliculée pour lutter contre la soumission chimique comprenant un principe actif et au moins 0,05 mg, de préférence de 0,2 à 5 mg, plus préférentiellement encore de 0,3 à 2mg d'au moins un agent colorant hydrosoluble choisi parmi l'indigocarmine, l'érythrosine, le brillant bleu FCF, l'alphazurine FG, le fast green FCF, la quinzarine green SS, l'orange II, la tartrazine, Sunset yellow FCF permettant la modification immédiate de la couleur d'une boisson dans laquelle est introduite ladite composition pharmaceutique solide non pelliculée.

Une telle forme pharmaceutique peut notamment être une forme orale choisie parmi les comprimés non enrobés tels que les comprimés classiques, les comprimés à sucer, les comprimés sublinguaux, les comprimés à croquer, les comprimés effervescents, les comprimés dispersibles, les comprimés orodispersibles ; une poudre pour sachets ou gélules, les films fins.

L'invention est plus particulièrement utile pour des compositions pharmaceutiques à libération immédiate, car le malfaiteur souhaitera que l'effet de perte de vigilance soit le plus rapide possible. Elle pourrait cependant être adaptée à des formes à libération contrôlée.

L'homme du métier sait adapter la formulation en fonction de la forme pharmaceutique et de la libération souhaitées.

Les excipients pharmaceutiquement acceptables mis en oeuvre dans les compositions pharmaceutiques solides non pelliculées selon l'invention sont des excipients classiquement utilisés.

On peut citer à titre d'exemples:
- les liants : par exemple les dérivés de la cellulose tels que l'HPMC, en particulier les grades Pharmacoat® 603 et Pharmacoat® 606, ou l'hydroxypropylcellulose ou l'hydroxyéthylcellulose, la cellulose microcristalline, les dérivés de la polyvinylpyrrolidone, en particulier le grade PVP K 30, les dérivés du polyéthylène glycol, en particulier le polyéthylène glycol dont le poids moléculaire est compris entre 600 et 7000, tels que le PEG4000 et le PEG6000 notamment, et leurs mélanges, des dérivés vinyliques tels que le polyvinylalcool;
- les diluants : par exemple les diluants solubles tels que le lactose, le mannitol, les dérivés de la cellulose telle que la cellulose microcristalline;
- les conservateurs : par exemple les parabens, les anti-oxydants tel que l'acide ascorbique;
- les solubilisants : par exemple les poloxamers, les cyclodextrines;
- les délitants : par exemple la crospovidone, la croscarmellose sodique;
- les édulcorants : comme l'aspartam, l'acesulfam potassique;
- les lubrifiants : le stéarate de magnésium, le stéarylfumarate de sodium, l'huile de coton;
- les arômes : tels que l'arôme menthe, citron, cerise noire, etc;
- les tensioactifs : les sels alcalins ou alcalinoterreux des acides gras, le sodium dodécyl sulfate et le docusate de sodium, les huiles polyoxyéthylénées, de préférence l'huile de ricin hydrogénée polyoxyéthylénée, les copolymères polyoxyéthylène- polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges ;
- les agents d'écoulement (glidants) : par exemple la silice, le talc, leur mélange.

Dans le cadre de la présente invention, on entend par comprimé orodispersible un « comprimé multiparticulaire se désagrégeant dans la bouche au contact de la salive en moins de 40 secondes». Selon un mode de réalisation particulier, l'invention porte sur un tel comprimé qui est à base d'un mélange d'excipients et de particules de principe actif enrobé présentant des caractéristiques intrinsèques de compression. La proportion de mélange d'excipients par rapport aux particules de principe actif enrobé est de 0,4 4 à 6, de préférence de 1 à 4 parties en poids. Le mélange d'excipients comprend :
- un agent de désagrégation ou délitant,
- un agent soluble diluant à propriétés liantes,
- un lubrifiant,
- éventuellement, un agent perméabilisant, des édulcorants, des arômes,
- un agent colorant permettant de lutter contre la soumission chimique,
- et éventuellement au moins un des composés permettant de lutter contre la soumission chimique choisi parmi les agents opacifiants, des les agents fluorescents, les particules flottantes, les particules perceptibles en bouche, et/ou les microgranules effervescents.

La proportion d'agent de désagrégation et d'agent soluble par rapport à la masse du comprimé étant de 1 à 15%, de préférence de 2 à 7% en poids pour le premier et de 30 à 90%, de préférence de 40 à 70% en poids pour le second.

L'agent soluble diluant à propriétés liantes est constitué par un polyol de moins de 13 atomes de carbone se présentant soit sous la forme du produit directement compressible dont le diamètre moyen des particules est compris entre 100 et 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, le sorbitol ne pouvant être utilisé seul.

L'agent de désagrégation est choisi dans le groupe comprenant notamment la carboxyméthylcellulose sodique réticulée désignée dans le métier par le terme croscarmellose, la crospovidone et leur mélange. Grâce au choix et à la proportion de cet agent de désagrégation, le comprimé conserve une dureté acceptable pour des conditions de manipulation normales des comprimés lorsqu'ils sont conservés en conditionnement étanche jusqu'à des températures d'au moins 30° C.

Le lubrifiant préférentiellement utilisé dans ce mélange d'excipients est choisi dans le groupe comprenant le stéarate de magnésium, le stéarylfumarate de sodium, l'acide stéarique, le polyoxyéthylèneglycol micronisé (Macrogol 6000 micronisé), et leurs mélanges Il peut être utilisé dans une proportion de 0,05 à 2% par rapport à la masse totale du comprimé.

Comme agent perméabilisant on utilise un composé choisi dans le groupe comprenant notamment des silices ayant une grande affinité pour les solvants aqueux, telles que la silice précipitée plus connue sous le nom de marque Syloid, les maltodextrines, les 1-cyclodextrines et leurs mélanges.

L'agent perméabilisant permet la création d'un réseau hydrophile qui facilite la pénétration de la salive et contribue ainsi à une meilleure désagrégation du comprimé.

Les différents composés et procédés de fabrication des comprimés orodispersibles décrits dans FR2785538, WO0027357 FR2679451, WO 93/01805, FR2766089, WO 00/51568, FR2790387, WO 03/039520, FR2831820 peuvent être mis en oeuvre dans la présente invention.

L'invention va être décrite de façon plus détaillée ci-après, notamment à l'aide des exemples qui sont donnés à titre d'illustration seulement.

### EXEMPLES

### Exemple 1

On prépare des comprimés orodispersibles comprenant 5 mg de zolpidem présentant la composition suivante :

| constituants | % | mg/unité |
|---|---|---|
| Grains Zolpidem | 32,8 | 41,05 |
| Cellulose microcristalline | 10,0 | 12,50 |
| Mannitol | 43,7 | 54,57 |
| Crospovidone | 10,0 | 12,50 |
| Colorant E132 | 0,4 | 0,50 |
| Aspartam | 1,0 | 1,25 |
| Arôme | 0,1 | 0,13 |
| Silice | 1,0 | 1,25 |
| Mg stéarate | 1,0 | 1,25 |
| *Total* | *100,0* | *125,0* |

Les comprimés orodispersibles sont préparés de la façon suivante.

On prépare dans un premier temps les grains de zolpidem qui présentent la composition centésimale suivante :

| | |
|---|---|
| NPTAB 190 (180-220µm) | 56 |
| Zolpidem tartrate | 13 |
| Hypromellose 603 | 7 |
| HCl1N | 2 |
| Aquacoat ECD30 | 13 |
| Hypromellose 603 | 6 |
| Triéthyl citrate | 3 |

On dissout le zolpidem tartrate dans de l'eau à l'aide d'HCl, puis on prépare une dispersion par ajout d'hypromellose 603. Dans un lit fluidisé GPCG1 (Glatt), on introduit des sphères de sucre NPTAB 190 et la dispersion préparée ci-dessus. On introduit ensuite une dispersion aqueuse d'aquacoat ECD30, triéthyl citrate et hypromellose 603 pour obtenir un enrobage de masquage de goût.

Les grains de zolpidem sont ensuite mélangés aux excipients de compression. Le mélange pulvérulent est ensuite comprimé sur comprimeuse rotative (SVIAC PR12) dotée de poinçons ronds, convexe, à une force de compression de 5kN

On obtient des comprimés de 125mg de diamètre 7mm qui présentent les caractéristiques suivantes:
Dureté 24N
Désagrégation (mesurée selon monographie 2.9.1 de la pharmacopée européenne 6.1): 15 sec
Friabilité (mesurée selon monographie 2.9.7 de la pharmacopée européenne 6.1): 0,03%.

Les comprimés sont agréables en bouche.

Un comprimé est introduit dans un récipient transparent contenant 250mL d'eau. Une coloration bleu intense apparait dès que le comprimé est désagrégé.

Un second comprimé est introduit dans un récipient transparent contenant 250mL de jus d'orange. La couleur orange du jus se transforme immédiatement en une couleur intense verdâtre.

### Exemple 2 (selon un mode de réalisation non revendiqué)

On prépare un comprimé classique à libération immédiate comprenant 10 mg de Zolpidem.

| | % | Mg/unité |
|---|---|---|
| Grains Zolpidem | 32,8 | 82,0 |
| Cellulose microcristalline | 10,0 | 25,0 |
| Lactose | 32,7 | 81,75 |
| Calcium silicate | 20,0 | 50,0 |
| Povidone | 3, 0 | 7,5 |
| Silice | 0, 9 | 2,25 |
| Colorant E132 | 0,1 | 0,25 |
| Mg stéarate | 0,5 | 1,25 |
| *Total* | *100,0* | *250,0* |

On prépare dans un premier temps les grains de zolpidem. Les grains de zolpidem sont préparés comme dans l'exemple 1.

Les grains de Zolpidem sont ensuite mélangés avec les excipients mentionnés dans le tableau ci-dessus. Le mélange pulvérulent est alors comprimé.

Un comprimé est alors dissous dans un verre de jus d'orange sans pulpe. Aussitôt après introduction et agitation de la forme pharmaceutique, une coloration verdâtre et un trouble apparaissent de façon visible à l'oeil nu.

### Exemple 3

On prépare deux types de comprimés orodispersibles comprenant chacun 10 mg de Zolpidem présentant la formule suivante:

| constituants | % | mg/unité |
|---|---|---|
| Grains Zolpidem | 32,8 | 82,1 |
| Cellulose microcristalline | 9, 6 | 23, 90 |
| Mannitol | 30,0 | 75, 00 |
| Crospovidone | 5,0 | 12,50 |
| Particules flottantes | 20,0 | 50,00 |
| Colorant E132 | 1,0 | 2,50 |
| Arôme | 0,1 | 0,25 |
| Silice | 1,0 | 2,50 |
| Mg stéarate | 0,5 | 1,25 |
| *Total* | *100,0* | *250*,*0* |

Ces comprimés sont préparés comme dans l'exemple 1, en utilisant les constituants du tableau ci-dessus.

Pour la première série de comprimés (C1) les particules flottantes sont préparées de la manière suivante:
Des neutres NPTAB 190 (180-220µm) sont enrobés avec une dispersion aqueuse d'éthylcellulose, triacétine et talc. Le facteur d'enrobage est de 30% de masse sèche et le ratio talc/polymère est de 1 :2.

Pour la seconde série de comprimés (C2) les particules flottantes sont des particules de phosphate de calcium phosphate dibasique dihydrate enrobées par glycéryl palmitostéarate. Le rapport glycéryl palmitostéarate / phosphate de calcium est 1 :4.

Les comprimés des deux séries se désintègrent en moins de 30s, et présentent une sensation agréable en bouche.

Un comprimé de chaque type est introduit dans un verre d'eau. La désintégration se fait immédiatement et l'eau se colore en bleu intense et la présence de particules à la surface est détectable à l'oeil nu. Ces particules flottantes sont visibles à la surface pendant plus de 3 heures.

### Exemple 4

On prépare un comprimé orodispersible comprenant 10 mg de Zolpidem, des particules flottantes et un agent colorant présentant la formule suivante:

| | **mg/unité** | **%** |
|---|---|---|
| Grains enrobés Zolpidem Tartrate* | 80.00 | 17.8 |
| Avicel PH 200 | 45.00 | 10.0 |
| Mannitol SD 200 | 65.05 | 14.5 |
| **Particules flottantes** | **200.00** | **44.4** |
| Kollidon CL | 45.00 | 10.0 |
| Arôme blackcherry | 0.45 | 0.10 |
| Aspartam | 4.50 | 1.00 |
| **Sunset Yellow** (**E110**) | **1.00** | **0.22** |
| Syloïd 244 FP | 4.50 | 1.00 |
| Stéarate Mg | 4.50 | 1.00 |
| Total | 450.0 | 100.0 |

Ces comprimés sont préparés comme dans l'exemple 1, en utilisant les constituants du tableau ci-dessus.

Les particules flottantes sont préparées de la manière suivante : des neutres NPTAB 190 (180-220µm) sont enrobés avec une dispersion aqueuse d'éthylcellulose et de myvacet 9-45. Le facteur d'enrobage est de 30% de masse sèche et le ratio plastifiant/polymère est de 24%.

Après introduction et agitation dans un verre d'eau, la forme colore le milieu en jaune orangé et libère des particules flottantes visibles à la surface pendant plus de trois heures.

### Exemple 5 (selon un mode de réalisation non revendiqué)

On prépare un comprimé classique comprenant des particules flottantes à base de microgranules de cire de carnauba et un agent colorant.

| | **mg/unité** | % |
|---|---|---|
| Oxycodone HCl granulé | 10.89 | 4.36 |
| Avicel PH 102 | 25.00 | 10.00 |
| Mannitol SD 200 | 133.11 | 53.24 |
| **Pellets cire carnauba** | **50.00** | **20.0** |
| Starch 1500 | 25.00 | 10.00 |
| **Indigocarmin E132** | **1.00** | **0.40** |
| Syloïd 244 FP | 2.50 | 1.00 |
| Stéarate Mg | 2.50 | 1.00 |
| Total | 250.0 | 100.0 |

L'oxycodone est granulé avec 4,1% d'HPMC 603 en mélangeur granulateur à haut cisaillement. L'actif est ensuite mélangé avec les excipients de compression de la formule ci-dessus. Le mélange est ensuite comprimé sur comprimeuse rotative (SVIAC PR12) dotée de poinçons ronds, convexe, à une force de compression de 16kN

On obtient des comprimés de 250mg de diamètre 8.5mm qui présentent les caractéristiques suivantes:
Dureté 95N
Désagrégation (mesurée selon monographie 2.9.1 de la pharmacopée européenne 6.1): 3 min
Friabilité (mesurée selon monographie 2.9.7 de la pharmacopée européenne 6.1): 0,1%.

Les comprimés obtenus après introduction dans une boisson développent immédiatement une coloration bleue uniforme, et libère des particules flottantes visibles en surface pendant plus de 3 heures.

### Exemple 6

On prépare un comprimé orodispersible comprenant 5mg d'oxycodone HCl anhydre et un agent colorant.

| | **%** | **mg/unité** |
|---|---|---|
| Grains Oxycodone HCl* | **22.0** | **29.65** |
| Avicel PH 102 | 10.0 | 13.50 |
| Mannitol SD 200 | **53.4** | **72.09** |
| Crospovidone | 10.0 | 13.50 |
| **Indigocarmin E132** | **0.4** | **0.54** |
| Aspartam | 2.0 | 2.70 |
| Arôme | 0.50 | 0.675 |
| Syloïd 244 FP | 0.50 | 0.675 |
| Stéarate Mg | 1.25 | 1.69 |
| Total | 100.0 | 135.00 |

Les comprimés orodispersibles sont préparés de la façon suivante.

On prépare dans un premier temps les grains d'oxycodone qui présentent la composition centésimale suivante :

| **Grains Oxycodone HCl** | **%** | **mg/unité** |
|---|---|---|
| NPTAB 250 | **54.0** | **16.01** |
| Oxycodone HCl | 18.3 | 5.43 |
| Hypromellose 603 | **7.6** | **2.25** |
| Eudragit NE30D vs | 16.7 | 4.95 |
| Syloid 244FP | 3.4 | 1.01 |
| Total | 100.0 | 29.65 |

On dissout l'oxycodone dans de l'eau, puis on prépare une dispersion par ajout d'hypromellose 603. Dans un lit fluidisé GPCG1 (Glatt), on introduit des sphères de sucre NPTAB 250 sur lesquelles on pulvérise la dispersion préparée ci-dessus. On pulvérise ensuite une dispersion aqueuse d'Eudragit NE30D et Syloid pour obtenir un enrobage de masquage de goût.

Les grains d'oxycodone sont ensuite mélangés aux excipients de compression. Le mélange pulvérulent est ensuite comprimé sur comprimeuse rotative (SVIAC PR12) dotée de poinçons ronds, convexes, de diamètre 7mm. On obtient des comprimés de 135mg. Dès l'introduction d'un comprimé dans un verre d'eau suivi d'une agitation, une coloration bleue intense et uniforme se développe.

### Exemple 7 (selon un mode de réalisation non revendiqué)

On prépare un comprimé classique comprenant 10mg d'oxycodone HCl anhydre et un agent colorant

| | **mg/unité** | **%** |
|---|---|---|
| Oxycodone HCl granulé | 10.89 | 5.45 |
| Avicel PH 102 | 20.00 | 10.00 |
| Mannitol SD 200 | 144.12 | 72.06 |
| Starch 1500 | 20.00 | 10.00 |
| **Indigocarmin E132** | **1.0** | **0.50** |
| Syloïd 244 FP | 2.0 | 1.00 |
| Stéarate Mg | 2.0 | 1.00 |
| Total | 200.0 | 100.0 |

L'oxycodone granulé est préparé comme dans l'exemple 5. Il est ensuite mélangé aux excipients de compression pour obtenir sur presse rotative des comprimés de 200mg de diamètre 8mm. Ce comprimé dissous dans un verre de jus de pomme développe une coloration verdâtre visible en moins d'une minute.

### Exemple 8 (selon un mode de réalisation non revendiqué)

On prépare un comprimé classique comprenant 10 mg de Zolpidem, un agent colorant et un agent opacifiant.

| | **mg/unité** | **%** |
|---|---|---|
| Zolpidem tartrate | 10.00 | 4.0 |
| Avicel PH 200 | 25.00 | 10.0 |
| Lactose DCL 21 | 152.75 | 61.1 |
| **Calcium silicate (FM 1000)** | **50.00** | **20.0** |
| PVP K30 | 7.50 | 3.0 |
| **Colorant E132** | **1.00** | **0.4** |
| Syloïd 244 FP | 2.50 | 1.0 |
| Stéarate Mg | 1.25 | 0.5 |
| Total | 250.00 | 100.0 |

Dans cet exemple, l'actif est mélangé directement à l'état poudre avec les excipients de compression. Le mélange permet d'obtenir, sur presse rotative dotée de poinçons ronds de diamètre 8.5mm, des comprimés de 250 mg. Après introduction et agitation d'un comprimé dans un verre d'eau, une coloration bleue et un trouble visible à l'oeil nu apparaissent en moins d'une minute.

### Exemple 9

### Grains montés de Zolpidem

On dissout le zolpidem tartrate dans de l'eau à l'aide d'HCl, puis on prépare une dispersion par ajout d'hypromellose 603.
Les grains de Zolpidem sont obtenus par pulvérisation de la dispersion sur les sphères de sucre NPTAB190 au sein d'un lit fluidisé.

| | % | Mg/unité |
|---|---|---|
| NPTAB 190 (180-220µm) | 73.19 | 22.52 |
| Zolpidem tartrate | 16.25 | 5.00 |
| Hypromellose 603 | 8.93 | 2.75 |
| HCl1N | 1.64 | 0.50 |
| total | 100.0 | 30.77 |

### Coloration des grains montés de Zolpidem

Une dispersion d'Aquacoat est ensuite préparée avec l'HPMC, le TEC (triethylcitrate) et le colorant ; elle est pulvérisée sur les grains montés en actif au sein d'un lit fluidisé.

| | % grains enrobés | mg par unité |
|---|---|---|
| **grains montés Zolpidem** | 75, 97 | 30,77 |
| Aquacoat ECD30 | 10, 17 | 4,12 |
| HPMC 603 | 10, 17 | 4,12 |
| TEC | 2,44 | 0, 99 |
| Colorant E132 | 1,24 | 0,50 |
| Total | 100,00 | 40,51 |

### Coloration des particules flottantes

Les particules flottantes colorées sont préparées de la manière suivante : des neutres NPTAB 250 sont enrobés avec une dispersion aqueuse d'éthylcellulose et de Myvacet® 9-45 contenant le colorant solubilisé par pulvérisation dans un lit fluidisé.

| | % grains enrobés | mg/gélule |
|---|---|---|
| **NPTAB 250 (200-300µm)** | 79,33 | 79,33 |
| Aquacoat ECD 30D | 15, 87 | 15, 87 |
| Myvacet^{®} 9-45 | 3, 81 | 3, 81 |
| Indigocarmine E132 | 1,00 | 1,00 |
| Total Mis en OEuvre | 100,00 | 100,00 |

Les deux populations sont mélangées dans les proportions : 40.51 mg de particules colorées de zolpidem et 100mg de particules flottantes colorées. Les deux populations ne sont pas différentiables. Lorsque la gélule est ouverte et son contenu introduit dans un verre d'eau, la coloration bleue et les particules flottantes apparaissent aussitôt.

## Revendications

1. Méthode permettant la détection immédiate d'un principe actif qui modifie l'état de conscience d'une victime introduit illicitement dans une boisson, ladite méthode comprenant :
* la mise en solution dans une boisson d'une forme pharmaceutique qui consiste en :
- un principe actif qui modifie l'état de conscience d'une victime,
- au moins 0,05 mg, de préférence de 0,2 à 5 mg, plus préférentiellement encore de 0,3 à 2 mg, d'un agent colorant hydrosoluble choisi parmi l'indigocarmine, l'érythrosine, le brillant bleu FCF, l'alphazurine FG, le fast green FCF, la quinzarine green SS, l'orange II, la tartrazine, Sunset yellow FCF,
- des particules perceptibles en bouche et flottantes présentant une densité inférieure à 1 et un diamètre supérieur à 500 µm, lesdites particules étant des microgranules comprenant un support neutre insoluble ou rendu insoluble dans l'eau ou dans une solution alcoolique par enrobage d'une matière lipidique, ou par enrobage avec un polymère insoluble,
- au moins un excipient pharmaceutiquement acceptable, ladite forme pharmaceutique étant une forme pharmaceutique solide non pelliculée se présentant sous forme de comprimé à croquer ou de comprimé orodispersible ;
* la détection de la forme pharmaceutique dans ladite boisson par la modification immédiate des propriétés organoleptiques de la boisson, la modification des propriétés organoleptiques se produisant en moins d'une minute, de préférence en moins de 30 secondes, plus préférentiellement encore en moins de 15 secondes à partir de l'introduction de la forme pharmaceutique dans la boisson.

2. Méthode selon la revendication 1, **caractérisée en ce que** le principe actif qui modifie l'état de conscience d'une victime est monté sur les particules perceptibles en bouche.

3. Méthode selon la revendication 1 ou 2, **caractérisée en ce que** le principe actif qui modifie l'état de conscience d'une victime est choisi dans le groupe comprenant : les anxyolytiques, les hypnotiques, les sédatifs, les analgésiques.

4. Méthode selon l'une quelconque des revendications 1 à 3, **caractérisée en ce que** les particules perceptibles en bouche sont présentes dans la forme pharmaceutique en une quantité d'au moins 25 mg, de préférence 40 mg.

5. Méthode selon l'une quelconque des revendications 1 à 4, **caractérisée en ce que** l'excipient pharmaceutiquement acceptable est choisi dans le groupe comprenant des liants, des diluants, des conservateurs, des solubilisants, des délitants, des édulcorants, des lubrifiants, des arômes, des tensioactifs, des agents d'écoulement, et leurs mélanges.

6. Forme pharmaceutique solide non pelliculée permettant la détection immédiate d'un principe actif qui modifie l'état de conscience d'une victime introduit illicitement dans une boisson, ladite forme pharmaceutique consistant en :
- un principe actif qui modifie l'état de conscience d'une victime,
- au moins 0,05 mg, de préférence de 0,2 à 5 mg, plus préférentiellement encore de 0,3 à 2 mg, d'un agent colorant hydrosoluble choisi parmi l'indigocarmine, l'érythrosine, le brillant bleu FCF, l'alphazurine FG, le fast green FCF, la quinzarine green SS, l'orange II, la tartrazine, Sunset yellow FCF,
- des particules perceptibles en bouche et flottantes présentant une densité inférieure à 1 et un diamètre supérieur à 500 µm, lesdites particules étant des microgranules comprenant un support neutre insoluble ou rendu insoluble dans l'eau ou dans une solution alcoolique par enrobage d'une matière lipidique, ou par enrobage avec un polymère insoluble,- et au moins un excipient pharmaceutiquement acceptable,
ladite forme pharmaceutique se présentant sous forme de comprimé à croquer ou de comprimé orodispersible,
ladite forme pharmaceutique permettant la détection du principe actif illicitement introduit dans la boisson en moins d'une minute, de préférence en moins de 30 secondes, plus préférentiellement encore en moins de 15 secondes à partir de l'introduction de ladite forme pharmaceutique dans ladite boisson.

7. Forme pharmaceutique selon la revendication 6, **caractérisée en ce que** le principe actif qui modifie l'état de conscience d'une victime est monté sur les particules perceptibles en bouche.

8. Forme pharmaceutique selon la revendication 6 ou 7, **caractérisée en ce qu'**il s'agit d'un comprimé orodispersible comprenant au moins un principe actif enrobé et **en ce que** l'excipient est un mélange d'excipients comprenant:
- un agent de désagrégation,
- un agent soluble diluant à propriétés liantes,
- un lubrifiant,
- éventuellement un agent perméabilisant, des édulcorants, des arômes.

## Patentansprüche

1. Verfahren, das den sofortigen Nachweis eines unerlaubterweise einem Getränk zugegeben Wirkstoffs, der auf ein Opfer bewusstseinsverändernd wirkt, ermöglicht, wobei besagtes Verfahren umfasst:
* das Auflösen einer pharmazeutischen Darreichungsform in einem Getränk umfassend:
- ein Wirkstoff, der auf ein Opfer bewusstseinsverändernd wirkt,
- mindestens 0,05 mg, bevorzugt 0,2 bis 5 mg, noch mehr bevorzugt 0,3 bis 2 mg, eines wasserlöslichen Farbstoffes der ausgewählt ist aus Indigokarmin, Erythrosin, Brillantblau FCF, Alphazurine FG, Fast Green FCF, Quinzarine Green SS, Orange II, Tartrazin, Sunsetgelb FCF,
- im Mund wahrnehmbare und aufschwebende Teilchen die eine kleinere Dichte als 1 und einen größeren Durchmesser als 500 µm aufweisen, wobei besagte Teilchen Mikrogranulate sind umfassend einen neutralen Träger der unlöslich ist oder für in Wasser oder einer alkoholischen Lösung unlöslich gemacht wurde durch Umhüllung mit einer Lipidsubstanz oder durch Umhüllung mit einem unlöslichen Polymer,
- mindestens einem pharmazeutisch unbedenklichen sonstigen Bestandteil, wobei besagte pharmazeutische Darreichungsform eine feste unbeschichtete pharmazeutische Darreichungsform ist vorliegend als kaubare Tablette oder im Mund zerfallende Tablette;
*der Nachweis der pharmazeutischen Darreichungsform in besagtem Getränk durch die sofortige Veränderung der organoleptischen Eigenschaften des Getränks, wobei die Veränderung der organoleptischen Eigenschaften in weniger als einer Minute, bevorzugt in weniger als 30 Sekunden, und noch mehr bevorzugt in weniger als 15 Sekunden ab Zugabe der pharmazeutischen Darreichungsform in das Getränk erfolgt.

2. Verfahren nach Anspruch 1, **dadurch gekennzeichnet, dass** der Wirkstoff, der auf ein Opfer bewusstseinsverändernd wirkt, aufgebracht ist auf die im Mund wahrnehmbaren Teilchen.

3. Verfahren nach Anspruch 1 oder 2, **dadurch gekennzeichnet, dass** der Wirkstoff, der auf ein Opfer bewusstseinsverändernd wirkt, ausgewählt ist aus der Gruppe enthaltend: Anxiolytika, Hypnotika, Sedativa, Analgetika.

4. Verfahren nach irgendeinem der Ansprüche 1 bis 3, **dadurch gekennzeichnet, dass** die im Mund wahrnehmbaren Teilchen in der pharmazeutischen Darreichungsform in einer Menge von mindestens 25 mg, bevorzugt 40 mg vorliegen.

5. Verfahren nach einem beliebigen der Ansprüche 1 bis 4, **dadurch gekennzeichnet, dass** der pharmazeutisch unbedenkliche sonstige Bestandteil ausgewählt ist aus der Gruppe enthaltend Bindemittel, Verdünnungsmittel, Konservierungsmittel, Solubilisierungsmittel, Sprengmittel, Süßstoffe, Schmiermittel, Aromastoffe, Tenside, Fließregulierungsmittel und deren Mischungen.

6. Feste unbeschichtete pharmazeutische Darreichungsform, die den sofortigen Nachweis eines, unerlaubterweise einem Getränk zugegebenen Wirkstoffs, der auf ein Opfer bewusstseinsverändernd wirkt, ermöglicht, besagte pharmazeutische Darreichungsform bestehend aus:
- ein Wirkstoff, der auf ein Opfer bewusstseinsverändernd wirkt,
- mindestens 0,05 mg, bevorzugt 0,2 bis 5 mg, noch mehr bevorzugt 0,3 bis 2 mg, eines wasserlöslichen Farbstoff der ausgewählt ist aus Indigokarmin, Erythrosin, Brillantblau FCF, Alphazurine FG, Fast Green FCF, Quinzarine Green SS, Orange II, Tartrazin, Sunsetgelb FCF,
- im Mund wahrnehmbare und aufschwebende Teilchen die eine Dichte von kleiner als 1 und einen Durchmesser von größer als 500 µm aufweisen, wobei besagte Teilchen Mikrogranulate sind umfassend einen neutralen Träger der unlöslich ist oder für in Wasser oder einer alkoholischen Lösung unlöslich gemacht wurde durch Umhüllung mit einer Lipidsubstanz oder durch Umhüllung mit einem unlöslichen Polymer,- und mindestens einem pharmazeutisch unbedenklichen sonstigen Bestandteil,
wobei besagte pharmazeutische Darreichungsform vorliegt als kaubare Tablette oder im Mund zerfallende Tablette,
wobei besagte pharmazeutische Darreichungsform den Nachweis eines unerlaubterweise einem Getränk zugegebenen Wirkstoffs in weniger als einer Minute, bevorzugt in weniger als 30 Sekunden, noch mehr bevorzugt in weniger als 15 Sekunden ab Zugabe der genannten festen pharmazeutischen Darreichungsform in das genannte Getränk ermöglicht.

7. Feste pharmazeutische Darreichungsform nach Anspruch 6, **dadurch gekennzeichnet, dass** der Wirkstoff, der auf ein Opfer bewusstseinsverändernd wirkt, auf die im Mund wahrnehmbaren Teilchen aufgebracht ist.

8. Feste pharmazeutische Darreichungsform nach Anspruch 6 oder 7, **dadurch gekennzeichnet, dass** es sich um im Mund zerfallende Tabletten handelt enthaltend mindestens einen umhüllten Wirkstoff und dass der sonstige Bestandteil eine Mischung aus sonstigen Bestandteilen ist enthaltend:
- ein Sprengmittel
- ein Verdünnungsmittel mit bindenden Eigenschaften
- ein Schmiermittel
- gegebenenfalls ein Permeabilisierungsmittel, Süßstoffe, Aromastoffe

## Claims

1. A method for the immediate detection of an active principle which modifies the state of consciousness of a victim illicitly introduced into a drink, said method comprising:
* dissolution in a drink of a pharmaceutical form consisting of:
- an active principle which modifies the state of consciousness of a victim,
- at least 0.05 mg, preferably from 0.2 to 5 mg, still more preferably from 0.3 to 2 mg of a watersoluble coloring agent selected from indigocarmine, erythrosine, brilliant blue FCF, alphazurine FG, fast green FCF, quinzarin green SS, orange II, tartrazine and sunset yellow FCF,
- particles perceptible in the mouth and floating having a density less than one and a diameter greater than 500 µm, said particles being microgranules comprising a blank support which is insoluble or rendered insoluble in water or in an alcoholic solution by coating with a lipid material or by coating with an insoluble polymer,
- at least one pharmaceutically acceptable excipient,
said pharmaceutical form being a solid non-film-coated pharmaceutical form being presented in the form of chewable tablet or orodispersible tablet;
* detection of the pharmaceutical form in said drink through the immediate modification of the organoleptic properties of the drink, the modification of the organoleptic properties taking place in less than one minute, preferably in less than 30 seconds, still more preferably in less than 15 seconds from the introduction of the pharmaceutical form in the drink.

2. The method according to claim 1, **characterized in that** the active principle which modifies the state of consciousness of a victim is coated onto the particles perceptible in the mouth.

3. The method according to claim 1 or claim 2, **characterized in that** the active principle which modifies the state of consciousness of a victim is selected from the group comprising: anxiolytics, hypnotics, sedatives and analgesics.

4. The method according to any one of claims 1 to 3, **characterized in that** the particles perceptible in the mouth are present in the pharmaceutical form in a quantity of at least 25 mg, preferably 40 mg.

5. The method according to any one of claims 1 to 4, **characterized in that** the pharmaceutically acceptable excipient is selected from the group comprising binders, diluents, preservatives, solubilizers, disintegrants, sweeteners, lubricants, flavors, surfactants, glidants and their mixtures.

6. A non-film-coated solid pharmaceutical form for the immediate detection of an active principle which modifies the state of consciousness of a victim illicitly introduced into a drink, said pharmaceutical form consisting of:
- an active principle which modifies the state of consciousness of a victim,
- at least 0.05 mg, preferably from 0.2 to 5 mg, still more preferably from 0.3 to 2 mg of a watersoluble coloring agent selected from indigocarmine, erythrosine, brilliant blue FCF, alphazurine FG, fast green FCF, quinzarin green SS, orange II, tartrazine and sunset yellow FCF,
- particles perceptible in the mouth and floating having a density less than one and a diameter greater than 500 µm, said particles being microgranules comprising a blank support which is insoluble or rendered insoluble in water or in an alcoholic solution by coating with a lipid material or by coating with an insoluble polymer,
- and at least one pharmaceutically acceptable excipient,
said pharmaceutical form being presented in the form of chewable tablet or orodispersible tablet,
said pharmaceutical form enabling the detection of the active principle illicitly introduced into the drink in less than one minute, preferably in less than 30 seconds, still more preferably in less than 15 seconds from the introduction of the pharmaceutical form in the drink.

7. The pharmaceutical form according to claim 6, **characterized in that** the active principle which modifies the state of consciousness of a victim is coated onto the particles perceptible in the mouth.

8. The pharmaceutical form according to claim 6 or claim 7, **characterized in that** it is an orodispersible tablet comprising at least a coated active principle and **in that** the excipient is a mixture of excipients comprising:
- a disintegrating agent,
- a soluble diluent with binding properties,
- a lubricant,
- possibly a permeabilizing agent, sweeteners and flavors.
